(19) Europäisches Patentamt
European Patent Office
Office européen des brevets

(11) Veröffentlichungsnummer: **0 304 781**
**A2**

# (12) EUROPÄISCHE PATENTANMELDUNG

(21) Anmeldenummer: **88113319.3**

(51) Int. Cl.⁴: **C07H 19/207 , C12M 1/40**

(22) Anmeldetag: **17.08.88**

(30) Priorität: **28.08.87 DE 3728772**

(43) Veröffentlichungstag der Anmeldung:
**01.03.89 Patentblatt 89/09**

(84) Benannte Vertragsstaaten:
**BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Bader, Hubert, Dr.**
**Im Münchfeld 23**
**D-6500 Mainz(DE)**
Erfinder: **Hoppe, Hans-Ullrich, Dr.**
**Habichtsweg 4**
**D-6238 Hofheim am Taunus(DE)**
Erfinder: **Magerstädt, Michael, Dr.**
**Schwedenstrasse 5**
**D-6237 Liederbach(DE)**
Erfinder: **Schlingmann, Merten, Prof. Dr.**
**Schneidhainer Strasse 32a**
**D-6240 Königstein/Taunus(DE)**
Erfinder: **Ulmschneider, Dieter, Dr.**
**Im Flemetz 6**
**D-6240 Königstein/Taunus(DE)**
Erfinder: **Walch, Axel, Dr.**
**Hans-Sachs-Strasse 5**
**D-6000 Frankfurt am Main 90(DE)**

## (54) Enzymreaktor mit polymerfixiertem Cofaktor.

(57) Verfahren zur Bindung von Cofaktoren an Polymere, bei dem man einen aromatische oder benzylische Aminogruppen tragenden Cofaktor zunächst in das entsprechende Isothiocyanat umwandelt und dieses dann an ein geeignete funktionelle Gruppen tragendes Polymeres anlagert.

EP 0 304 781 A2

## Enzymreaktor mit polymerfixiertem Cofaktor

Die Erfindung betrifft ein verbessertes Verfahren zur Fixierung der für enzymatische Reaktionen erforderlichen Cofaktoren an Polymere und einen Enzymreaktor, der neben einem oder mehreren Enzymen einen oder mehrere erfindungsgemäß polymerfixierte Cofaktoren enthält.

Es ist bekannt, daß enzymatische Reaktionen wegen ihrer hohen Selektivität und guten Ausbeuten bei der Herstellung wertvoller chemischer Verbindungen steigende Bedeutung erlangt haben. Dabei werden Enzyme nach verschiedenen Verfahren immobilisiert und dann als heterogene Katalysatoren angewandt. Sie können dann leicht aus dem Reaktionsgemisch abgetrennt und erneut eingesetzt werden.

Soweit Enzyme oder enzymatische Komplexe nur in Gegenwart eines niedermolekularen Cofaktors aktiv sind, stellte sich die Aufgabe, den Cofaktor ebenfalls zu immobilisieren, da er anderenfalls der Reaktionsmischung ständig neu zugesetzt werden müßte. Ein leicht löslicher, niedermolekularer Cofaktor könnte vom Reaktionsprodukt nicht durch einfache Membranfiltration abgetrennt werden, was zum völligen Verlust des teuren Cofaktors bei gleichzeitiger Verunreinigung des erhaltenen Endprodukts führen würde. Deshalb sind viele Untersuchungen zur Immobilisierung solcher Cofaktoren auf Polymeren beschrieben worden (vgl. P.-O. Larsson und K. Mosbach, Biotechnol. Bioeng. 13 (1971), 393; J. Grunwald und Th. M.S. Chang, Biochem. Biophys. Res. Commun. 81 (1978), 565; DE-PS 26 31 045, DE-PS 28 41 414; DE-OS 29 30 987).

Cofaktoren mit einem Adeninringsystem wie Nicotinamidadenin-dinucleotid ($NAD^+$/NADH), Nicotinamid-adenindinucleotidphosphat ($NADP^+$/NADPH), Adenosinmonophosphat (AMP), Adenosindiphosphat (ADP), Adenosintriphosphat (ATP), Flavin-adenin-dinucleotid (FAD/$FADH_2$) und Coenzym A, die covalent an feste Träger oder an lösliche Polymere gebunden sind, werden bereits in großem Umfang verwendet. So werden an feste Träger gebundene Cofaktoren als Liganden bei der Affinitätschromatographie eingesetzt. Wasserlösliche Polymere mit covalent gebundenen Cofaktoren sind bei der Affinitätsverteilung sehr brauchbar. Cofaktoren wie $NAD^+$/NADH und $NADP^+$/NADPH lassen sich regenerieren und in enzymatischen Systemen unter Kreislaufbedingungen verwenden.

Bei der Herstellung von an Polymere gebundenen, ein Adeninringsystem enthaltenden Cofaktoren geht man so vor, daß man zuerst das N-Atom in 6-Stellung mit einer reaktiven Gruppe verbindet, die dann ihrerseits mit einer funktionellen Gruppe des Makromoleküls reagieren kann. Dabei setzt man zuerst das Adeninringsystem am N-(1)-Atom mit einem Alkylierungsmittel um, das eine weitere funktionelle Gruppe trägt, die die Umsetzung mit dem Makromolekül ermöglichen soll. Als Alkylierungsmittel kommen Halogencarbonsäuren wie Jodessigsäure, Epoxide wie 3,4-Epoxibuttersäure, Lactone wie Propionlacton oder Aziridine wie Ethylenimin in Betracht.

Im Fall von AMP, ADP und ATP folgt unmittelbar auf die Alkylierung eine Dimroth-Umlagerung zur N-(6)-Form. An die Dimroth-Umlagerung schließt sich dann die Umsetzung mit dem Makromolekül an. Im Fall von $NAD^+$ und $NADP^+$ wird vor der Dimroth-Umlagerung eine Reduktion und nach der Dimroth-Umlagerung eine Reoxidation des Nicotinamidringes durchgeführt.

Schon aus der DE-PS 28 41 414 ist jedoch bekannt, daß diese zur Fixierung von Cofaktoren an Polymeren notwendigen Reaktionsfolgen unbefriedigend sind, weil durch die Vielzahl der notwendigen Reaktionen Ausbeuteverminderungen eintreten und der teure Cofaktor sich nur in einer Menge von 12 bis 40 % so an das Polymere fixieren läßt. Zur Ausbeuteverbesserung ist deshalb dort vorgeschlagen worden, die in 1-Stellung alkylierten Adeninderivate erst nach der Addition an das Polymere einer Dimroth-Umlagerung zu unterwerfen. Hierdurch lassen sich zwar die Ausbeuteverluste vermindern, jedoch bleibt die Zahl der erforderlichen Reaktionsschritte die gleiche, und bei den $NAD^+$- und $NADP^+$-Derivaten müssen eine Reduktion und eine Reoxidation des Nicotinamidringes weiterhin durchgeführt werden.

Es stellte sich deshalb die Aufgabe, das Verfahren zur Bindung der Cofaktoren an Polymere zu vereinfachen, um Ausbeute- und Aktivitätsverluste der teuren Cofaktoren zu vermindern.

Erfindungsgemäß wird dieses Ziel dadurch erreicht, daß man aromatische oder benzylische Aminogruppen tragende Cofaktoren zunächst, z.B. durch Umsetzung mit Thiophosgen, in das entsprechende Isothiocyanat umwandelt und dieses dann an ein geeignetes, funktionelle Gruppen tragendes Polymeres anlagert. Setzt man beispielsweise als Cofaktor NADH und ein aminogruppentragendes Polymer ein, entstehen hierbei Thioharnstoffderivate der allgemeinen Formel

$$\mathrm{NAD} - \underset{\mathrm{H}}{\mathrm{N}} - \overset{\mathrm{S}}{\overset{\|}{\mathrm{C}}} - \underset{\mathrm{H}}{\mathrm{N}} - \mathrm{Polymer}\ .$$

Bei Verwendung eines Hydroxygruppen tragenden Polymeren entstehen die entsprechenden Thiocarbaminate. Die Herstellung von Thioharnstoffen und Thiocarbaminaten aus Isothiocyanaten und Amino- oder Hydroxygruppen tragenden Verbindungen ist Stand der Technik (vgl. L. Drobnica, P. Krisian, J. Augustin in "The Chemistry of Functional Groups" (Patai, Ed.) part 2: "Cyanates and their Derivatives", Wiley, New York, Chapter 22, 1003).

Das zur Bindung des Cofaktors auszuwählende Polymere sollte wasserlöslich sein, da bei der Bindung des Cofaktors an ein wasserunlösliches Polymeres die vollständige Reduktion des gebundenen $NAD^+$ nicht mehr möglich ist und dadurch erhebliche Aktivitätseinbußen auftreten (vgl. H.-L. Schmidt und G. Grenner, Eur. J. Biochem. 67, 295-302 (1976).

Für die Fixierung der Cofaktoren werden wasserlösliche Polymere bevorzugt, die primäre oder sekundäre Aminogruppen tragen. Besonders bewährt haben sich Copolymere aus Vinylamin und Vinylmethylacetamid oder Vinylmethylamin und Vinylmethylacetamid, wobei das Verhältnis der Monomeren zwischen 1 : 99 und 40 : 60 Gew.-% variiert werden kann.

Zur Herstellung dieser Copolymeren setzt man N-Vinylformamid oder N-Vinyl-N-methylformamid mit anderen wasserlöslichen N-Vinylamiden, wie N-Vinyl-N-methylacetamid oder N-Vinylpyrrolidon um und hydrolysiert in wäßriger Lösung die eingebauten N-Vinylformamid- oder N-Vinyl-N-methylformamidbausteine mit starken Säuren, bevorzugt Salzsäure, zu N-Vinylamin- oder N-Vinyl-N-methylaminkettengliedern. Infolge der sehr leichten Verseifbarkeit der Formamide im Gegensatz zu anderen Vinylamiden werden die anderen einpolymerisierten N-Vinylamide dabei nicht in nennenswertem Umfang hydrolysiert. Durch Ionenaustausch werden schließlich abgespaltene Ameisensäure und eingesetzte Salzsäure entfernt.

Durch Variation der einpolymerisierten N-Vinylformamid-oder N-Vinyl-N-methylformamidmenge läßt sich der Basenteil beliebig einstellen. Wird N-Vinylformamid zur Copolymerisation eingesetzt, so enthält das Endprodukt N-Vinylaminbausteine und bei der Copolymerisation von N-Vinyl-N-methylformamid enthält das Endprodukt N-Vinyl-N-methylaminkettenglieder. Es kann jedoch auch eine Terpolymerisation,z.B. von N-Vinylformamid, N-Vinyl-N-methylformamid und N-Vinyl-N-methylacetamid durchgeführt werden. Durch Verseifung dieser Polymeren ist der Zugang zu polymeren Trägersubstanzen mit primären und sekundären Vinylaminbausteinen, also unterschiedlicher Basenreaktivität möglich, die ebenfalls zur Fixierung von Cofaktoren geeignet sind.

Sehr ähnliche Polymere enthält man auch durch Teilverseifung von Poly-N-vinylformamid- oder Poly-N-vinyl-N-methylformamidhomopolymerisaten. Hier läßt sich durch Variation der Säuremenge und der Verseifungszeit ebenfalls der Basenanteil beliebig einstellen. Auch ist durch Copolymerisation von N-Vinylformamid und N-Vinyl-N-methylformamid und anschließende Teilverseifung die Synthese von polymeren wasserlöslichen Trägersubstanzen mit unterschiedlicher Basenreaktivität möglich.

Die Einstellung einer gewünschten mittleren Molekülgröße der basischen, wasserlöslichen Trägersubstanzen kann durch eine Reihe von Maßnahmen, wie Wahl der Polymerisationstemperatur, des Lösemittels und der Initiatorkonzentration, in einem großen Ausmaß (MG 10000 bis 500000) beeinflußt werden.

Ebenfalls gute Erfahrungen wurden mit dem partiell alkylaminsubstituierten $\alpha,\beta$-Poly-(2-hydroxyethyl)-D,L-aspartamid gemacht.

Es können jedoch auch andere wasserlösliche Polymere eingesetzt werden, die mit Isothiocyanaten reagierende funktionelle Gruppen tragen. Beispielsweise können auch Polyethylenimin oder wasserlösliche Kohlenhydrate verwendet werden.

Mit dem erfindungsgemäßen Verfahren gelingt es, Cofaktoren in praktisch quantitativer Ausbeute ohne Aktivitätsverlust an Polymere zu fixieren und dabei die bisher als notwendig angesehene, mehrstufige Reaktionsfolge zu vermeiden. Die so gewonnenen polymerfixierten Cofaktoren können in vielfältiger Weise eingesetzt werden. Sie können beispielsweise zusammen mit einem oder mehreren Enzymen in Mikrokapseln eingeschlossen werden. Die Herstellung derartiger Mikrokapseln mit einer semipermeablen Wandung, die den Durchtritt von Substrat und Reaktionsprodukt gestattet, das oder die Enzyme und den gebundenen Cofaktor aber zurückhält, ist seit langem Stand der Technik (vgl. Th. M.S. Chang, Biomedical Appl., Immobilized Enzyme Proteins, Vol. 1 (1977), 69, Plenum, New York 1977).

Die Enzyme können zusammen mit dem polymerfixierten Cofaktor auch in einem mit einer Ultrafiltrationsmembran ausgestatteten Membranreaktor verwendet werden, dessen Membran dazu dient, die eingesetzten Enzyme und den Cofaktor im Reaktor zurückzuhalten, aber das niedermolekulare Produkt und das nicht umgesetzte Substrat durchzulassen. Auch derartige Membranreaktoren sind bereits Stand der Technik und beispielsweise in der DE-OS 29 30 078 beschrieben.

Sowohl mit semipermeablen Mikrokapseln als auch in der Ultrafiltrationszelle läßt sich eine enzymatische Reaktion kontinuierlich durchführen, wenn der oder die Cofaktoren mehrere Reaktionen nacheinander katalysieren und dabei wieder in ihren Ausgangszustand überführt werden. Beispielsweise kann $NAD^+$ mit Hilfe der Malatdehydrogenase Malat in Oxalacetat umwandeln. Das dabei entstehende NADH kann dann mit

Hilfe der Alkoholdehydrogenase Acetaldehyd zum Ethanol reduzieren. Danach liegt wieder $NAD^+$ vor, das für eine erneute Reduktion zur Verfügung steht. Ein derartiges System regeneriert sich also ständig wieder und kann theoretisch diese Reaktion auf unbegrenzte Zeit durchführen. In der Praxis zeigt sich allerdings, daß diese aus mehreren Enzymen und Cofaktoren bestehenden Systeme allmählich ihre Aktivität verlieren. Wie die in den Beispielen näher beschriebenen Versuche verdeutlichen, ist jedoch bei den erfindungsgemäß hergestellten Enzymreaktoren ein Aktivitätsverlust nicht zu beobachten gewesen. Hinzu kommt, daß die erfindungsgemäße Fixierung des Cofaktors in einer einfachen Eintopfreaktion und in nahezu quantitativer Ausbeute verläuft.

**Beispiel 1**

**Darstellung von 6-Isothiocyanato-nicotinamidadenindinucleotid ("NAD-NCS")**

2 mmol $NAD^+$ wurden in einem Rundkolben mit aufgesetztem Blasenzähler in 10 ml $H_2O$ gelöst und mit wenig $Na_2CO_3$ versetzt. Unter starkem Rühren wurde bei 20° C eine Lösung von 20 mmol Thiophosgen in 10 ml $CHCl_3$ dazugegeben. Der pH wurde alle 10 Minuten kontrolliert und mit Zugabe kleiner Mengen Natriumcarbonat zwischen 5,5 und 8,5 gehalten. Nach 3,5 Stunden änderte sich der pH nicht mehr und es war keine weitere Gasentwicklung zu beobachten. Das Gemisch wurde trockengezogen und gefriergetrocknet. Das leicht bräunliche Produktgemisch konnte durch Säulenchromatographie auf Kieselgel mit Wasser/Acetonitril als Eluent von Nebenprodukten gereinigt werden. Es ist jedoch empfehlenswert, das Produktgemisch ungereinigt zur Umsetzung mit dem Polymer (s. Beispiel 2) einzusetzen, da eine Reinigung des polymerfixierten Produktes durch Ultrafiltration wesentlich einfacher ist und auch die Empfindlichkeit des NAD-NCS bei einer Reinigung zu Verlusten durch Dimerisierung und Trimerisierung führt.

**Beispiel 2**

**Umsetzung von Vinylamin/Vinylmethylacetamid-Copolymer (4,8:95;2 Gew.-%) mit 6-Isothiocyanato-nicotinamidadenindinucleotid (NAD-NCS)**

863 mg Polymer ( $\hat{=}$ 1 mmol $NH_2$-Gruppen) in 5 ml $H_2O$ wurden in einem 50 ml-Rundkolben bei 20° C mit 10 ml einer Lösung von 2 mmol NAD-NCS in 20 ml Wasser versetzt, der pH wurde mit einer 1 N NaOH auf 8 eingestellt und die Lösung 15 Stunden bei 20° C gerührt. Danach wurde die Lösung in einer Ultrafiltrationszelle durch eine Celluloseacetatmembran (Ausschlußgrenze: Molekulargewicht 5000) bei 3 bar Überdruck ultrafiltriert, wobei 10mal mit jeweils 50 ml Wasser aufgefüllt und wieder filtriert wurde. Das Retentat sowie die 10 Permeate wurden im UV-Spektrometer bei $\lambda$ = 257 nm (Absorptionsmessung) vermessen. Die Permeate zeigten eine mit fortschreitender Ultrafiltration kleiner werdende Absorption, d.h. abnehmenden $NAD^+$-Gehalt. Das Retentat enthielt 70 bis 90 % des eingesetzten $NAD^+$. Dieses Ergebnis konnte durch Absorptionsmessung einer eingewogenen, in Wasser gelösten Probe des gefriergetrockneten Retentats und Vergleich mit einer mit reinem $NAD^+$ erstellten Eichkurve bestätigt werden. Nach Gefriertrocknung ergab das Retentat 12 g Produkt (85 % bezogen auf das Polymer).

Im Standardtest der Fa. Boehringer Mannheim wurde das Produkte anstelle von $NAD^+$ als Cofaktor bei der Bestimmung von Malat mit Malatdehydrogenase, von Ethanol mit Alkoholdehydrogenase sowie von Lactat mit Lactatdehydrogenase eingesetzt. In allen Fällen war die Enzymaktivität und damit auch die Cofaktoraktivität unverändert beim Ersatz von $NAD^+$ durch eine äquivalente Menge des polymerfixierten $NAD^+$.

**Beispiel 3**

**Kopplung von $\alpha,\beta$-Poly-(2-hydroxyethyl)-D,L-aspartamid, partiell substituiert mit Aminoethylgruppen ($H_2N$-PHEA), über diese Aminogruppen an NAD-NCS**

Das $H_2N$-PHEA enthielt $10^{-4}$ mol/g Aminogruppen. Da unter den hier verwendeten Bedingungen vorrangig die Aminogruppen mit dem Isothiocyanat reagieren, wurde NAD-NCS stöchiometrisch zu dieser Aminogruppenzahl eingesetzt. 1,5 g Polymer ($1,5 \times 10^{-4}$ mol Aminogruppen) wurden in ca. 25 ml $H_2O$ gelöst und in einem 100 ml-Rundkolben bei 20 °C mit einer Lösung von $1,4 \times 10^{-4}$ mol NAD-NCS versetzt. Der pH wurde mit NaOH auf 8 eingestellt. Das Gemisch wurde bei 20 °C 20 h lang gerührt und dann bei 3 bar über eine Celluloseacetatmembran (Ausschlußgrenze: Molekulargewicht 5000) ultrafiltriert, wobei 6 mal mit jeweils 60 ml Wasser aufgefüllt und wieder filtriert wurde. Durch Vergleich der UV-Absorption der Permeate bzw. des Retentats bei 257 nm wurde der Gehalt des Produkts an $NAD^+$ bestimmt. Er betrug 80 % der eingesetzten Menge. Das Retentat wurde gefriergetrocknet, Gesamtausbeute 1,4 g (92 %), so daß sich eine effektive Konjugatioursausbeute von 87 % ergibt.

**Beispiel 4**

**Mikroverkapselung eines Enzymsystems mit polymerfixiertem NAD**

Als Enzymsystem wurde Malatdehydrogenase (MDH), in Verbindung mit Glutamat-Oxalacetat-Transaminase (GOT), analog dem von Boehringer Mannheim vertriebenen Malat-Test, eingesetzt. Hierbei wurde L-Malat durch MDH mit $NAD^+$ zu Oxalacetat, NADH und $H^+$ umgesetzt. Um das Gleichgewicht vom L-Malat weg zu verschieben, wurde durch GOT und Zugabe von Glutamat das Oxalacetat in L-Aspartat und $\alpha$-Ketoglutarat umgesetzt.

In einem typischen Versuch wurden 0,01 ml einer 5 mg/ml Lösung von MDH, 0,01 ml einer 2 mg/ml Suspension GOT (beide in Wasser) und 25,5 mg des in Beispiel 2 beschriebenen $NAD^+$-Polymers ( $\hat{=}$ 10 mg $NAD^+$) mit Wasser auf 1,42 ml aufgefüllt. 0,3 ml dieser Lösung wurden mit 1 g einer 2 %igen wäßrigen Lösung von Alginat 20/60 gemischt und mit einer Spritze und Kanüle (Innendurchmesser 0,2 mm) in einer 0,4 %ige wäßrige Lösung von Polylysin, mittleres Molekulargewicht 5800, gesprüht. Aus 931 mg versprühter Lösung wurden 558 mg Mikrokapseln erhalten, die nach 15 Minuten aus der Polylysinlösung entfernt (dekantiert) und danach mit 1 %iger wäßriger NaCl-Lösung gewaschen wurden. Danach wurden die Kapseln 1 Minute lang in eine 12,5 %ige Glutardialdehydlösung eingelegt und damit vernetzt. Die Kapseln wurden in einem 400 ml Becherglas in Wasser gelagert und durch Einblasen von Stickstoff gerührt. Nach 17 Stunden, 2 Tagen, 3 Tagen, 6 Tagen und 7 Tagen wurden Aliquots der überstehenden Lösung abgenommen und bei 257 nm wurde gegen eine $NAD^+$-Eichkurve die UV-Absorption der Lösung gemessen und damit der $NAD^+$-Gehalt der überstehenden Lösung (d.h. der $NAD^+$-Verlust aus den Kapseln) bestimmt. Dabei ergab sich folgendes:

| Zeit | $NAD^+$-Verlust aus Kapseln | |
|---|---|---|
| 17 h<br>2 Tage | 1,6 % )<br>1,7 % ) | einzelne mechanisch beschädigte Kapseln sind mit bloßem Auge sichtbar |
| 3 Tage<br>4 Tage<br>7 Tage | 0,8 %<br>0,8 %<br>0,4 % | |

Nach 9 Tagen wurden 44 Kapseln, entsprechend ca. $0,7 \bullet 10^{-3}$ mmol $NAD^+$, abgenommen und unter den im Malat-Test von Boehringer Mannheim beschriebenen Bedingungen zur Umsetzung von Malat eingesetzt. Die Mikrokapseln erreichten einen Umsatz von 0,004 mg aus einem Malatüberschuß mit einer äquivalenten Menge $NAD^+$ innerhalb von 30 Minuten (theoretischer Umsatz 0,003 mg L-Malat).

**Beispiel 5**

**Halbkontinuierlicher Enzymreaktor mit polymerfixiertem NAD, bei dem der Cofaktor im Kreislauf regeneriert wurde**

Als Enzymsystem wurde hierzu Formiatdehydrogenase (FDH) und Lactatdehydrogenase (LDH) eingesetzt, wobei Formiat mit $NAD^+$ durch FDH in $CO_2$ und NADH überführt und Pyruvat mit NADH durch LDH in Lactat überführt wurde. Pyruvat und Formiat wurden als Substrate im Überschuß eingesetzt, $CO_2$ entwich und Lactat wurde als einziges Produkt der Reaktion in Lösung bestimmt. Zur Retention der Enzyme und des NAD-Polymers wurde eine Ultrafiltrationsmembran aus Celluloseacetat benutzt (Ausschlußgrenze Molekulargewicht 5000). Die Reaktion wurde in einer Ultrafiltrationszelle durchgeführt, wobei das Reaktionsgemisch unter einem Druck von 2 bar stand und im fraktionsweise abgenommenen Permeat das Lactat bestimmt werden konnte.

Es wurden NAD-Polymer nach Beispiel 2 (entsprechend 0,021 mmol NAD), 0,1 mg LDH, 4 Units FDH, 0,26 mmol Pyruvat und 0,26 mmol Formiat in 3,7 ml Phosphatpuffer, pH 7,2, eingesetzt. Unter Rühren und bei 2 bar $N_2$-Überdruck wurden die Permeatfraktionen je 30 Minuten gesammelt. Nach jeder Fraktion wurde das Konzentrat mit Phosphatpuffer, pH 7,2 auf die ursprünglichen 3,7 ml aufgefüllt. Nach jeder 4. Fraktion wurden außerdem 0,26 mmol Pyruvat und 0,26 mmol Formiat zugegeben. Die Permeate wurden mit dem von Boehringer Mannheim vertriebenen Lactatbestimmungstest auf den Lactatgehalt untersucht. Über 22 Fraktionen erhielten wir pro Permeatfraktion (je 1 ml) eine Lactatausbeute von 3 bis $6 \bullet 10^{-4}$ mmol Lactat. Nach Unterbrechung und Weiterführung des Test nach 2 Tagen (Wochenende) konnte weiterhin derselbe konstante Lactatgehalt pro Permeatfraktion erhalten werden.

**Beispiel 6**

**A. Herstellung eines N-Vinylamin-N-vinyl-N-methylacetamidcopolymeren, N-Vinylaminanteil 4,8 Gew.-%**

36 g N-Vinyl-N-methylacetamid und 3 g N-Vinylformamid wurden in 40 ml Isopropanol mit 400 mg $\alpha,\alpha'$-Azo-isobutyronitril als Initiator bei 80° C (24 h) copolymerisiert.

Anschließend wurde das Isopropanol unter gleichzeitiger Wasserzugabe (40 ml) abdestilliert. Zu der entstandenen wäßrigen Polymerlösung fügte man 40 ml konz. Salzsäure und erhitzte 8 h bis 110° C. Man verdünnte die stark saure Reaktionsmischung mit Wasser und entfernte anschließend mit einem stark basischen Ionenaustauscher Salz- und Ameisensäure. Nach Einengung im Vakuum (~100 mbar) wurde eine wäßrige Lösung eines N-Vinylamin/N-Vinyl-N-methylacetamidcopolymeren mit einem N-Vinylaminanteil von ~ 4,8 Gew-% erhalten. Der Feststoffgehalt der Polymerlösung betrug 33,3%, der pH-Wert 11,5 und vom Polymeren wurde eine reduzierte Viskosität von 0,25 dl/g (gemessen 1 %ig in $H_2O$ bei 25° C) bestimmt.

**B. Herstellung eines N-Vinylamin/N-Vinylpyrrolidoncopolymeren, N-Vinylaminanteil 5,7 Gew-%**

20 g N-Vinylpyrrolidon und 2 g N-Vinylformamid wurden in 120 ml Wasser, dem 1 ml $NH_4$-OH-Lösung zugefügt war, bei 80° C und Argonüberlagerung polymerisiert. Initiator $\alpha,\alpha'$-Azo-iso-Butyronitril 110 mg, Polymerisationsdauer 24 h. Zu der entstandenen wäßrigen Copolymerlösung gab man 145 ml konz. Salzsäure und erhitzte 7 h unter Rückfluß (110° C). Anschließend wurde der Ansatz mit einem stark basischen Ionenaustauscher, wie bei A beschrieben, aufgearbeitet. Man erhielt nun 160 g wäßrige Lösung eines N-Vinylamin/N-vinylpyrrolidoncopolymeren mit einem N-Vinylaminanteil von 5,7 Gew-%. Der Feststoffgehalt der Polymerlösung war 9,53 %, der pH-Wert 10,1 und vom Polymeren wurde eine reduzierte Viskosität von 1,61 dl/g (gemessen 1 %ig in $H_2O$ bei 25° C) bestimmt.

**C. Herstellung von N-Vinyl-N-methylamin/N-Vinyl-N-methylformamidcopolymeren (nach Verfahren B)**

Man polymerisierte N-Vinyl-N-methylformamid mit α,α′-Azo-iso-butyronitril als Initiator und verseifte in wäßriger Lösung das Homopolymerisat mit Salzsäure bei 100 °C nur teilweise. Die Ergebnisse der Teilverseifung in Abhängigkeit von der Zeit und der Salzsäurekonzentration sind folgender Tabelle zu entnehmen:

| Verseifung | Verfahren (a) | Verfahren (b) |
|---|---|---|
| 1/2 Stunde<br>2 Stunden | 9,5 % basischer N<br>15,7 % basischer N | 13,5 % basischer N<br>20,1 % basischer N |
| Bei Verfahren (a) wird ein Mol HCl auf ein Mol N-Vinyl-N-methylformamid, bei Verfahren (b) 2,5 Mol HCl auf ein Mol eingesetzt. | | |

Die Bestimmung der basischen Stickstoffwerte erfolgte nach Entfernung von Ameisen- und Salzsäure mittels eines Ionenaustauschers; eine 100 %ige Verseifung entspricht einem basischen Stickstoffwert von 24,9 %. Bei 9,0 % bas. Stickstoff liegt beispielsweise ein N-Vinyl-N-methylamin-N-vinyl-N-methylformamidcopolymerisat mit einem N-Vinyl-N-methylaminanteil von 36,0 Gew-% vor.

**Ansprüche**

1. Verfahren zur Bindung von Cofaktoren an Polymere, dadurch gekennzeichnet, daß man einen aromatische oder benzylische Aminogruppen tragenden Cofaktor zunächst in das entsprechende Isothiocyanat umwandelt und dieses dann an ein geeignete funktionelle Gruppen tragendes Polymeres anlagert.

2. Verfahren nach Anspruch 1, dadurch gekennzeichnet, daß das Polymere wasserlöslich ist.

3. Verfahren nach Anspruch 1, dadurch gekennzeichnet, daß das wasserlösliche Polymere primäre oder sekundäre Aminogruppen trägt.

4. Verfahren nach Anspruch 3, dadurch gekennzeichnet, daß das wasserlösliche Polymere ein Copolymerisat aus Vinylamin und Vinylmethylacetamid oder ein Copolymerisat aus Vinylmethylamin und Vinylmethylacetamid ist, wobei das Verhältnis der Monomeren zwischen 1 : 99 und 40 : 60 Gew.-% liegen kann.

5. Verfahren nach Anspruch 3, dadurch gekennzeichnet, daß das wasserlösliche Polymere ein partiell alkylaminsubstituiertes α,β-Poly-(2-hydroxyethyl)-D,L-aspartamid ist.

6. Verfahren nach Anspruch 1, dadurch gekennzeichnet, daß das wasserlösliche Polymere Polyethylenimin oder ein wasserlösliches Kohlenhydrat ist.

7. Cofaktor, dadurch gekennzeichnet, daß er über eine aromatische oder benzylische Aminogruppe mittels einer Thioharnstoff oder Thiocarbaminatbrücke an ein wasserlösliches Polymeres gebunden ist.

8. Enzymreaktor, dadurch gekennzeichnet, daß er einen an ein Polymeres gebundenen Cofaktor gemäß Anspruch 7 und ein oder mehrere Enzyme enthält.

9. Enzymreaktor nach Anspruch 8, dadurch gekennzeichnet, daß der an das Polymere gebundene Cofaktor und die Enzyme in Mikrokapseln enthalten sind.

10. Enzymreaktor nach Anspruch 8, dadurch gekennzeichnet, daß der an das Polymere gebundene Cofaktor und die Enzyme in einem mit einer Ultrafiltrationsmembran ausgestatteten Membranreaktor enthalten sind.